(19) Europäisches Patentamt
European Patent Office
Office européen des brevets

(11) Veröffentlichungsnummer : **0 474 589 A1**

(12) **EUROPÄISCHE PATENTANMELDUNG**

(21) Anmeldenummer : **91810593.3**

(22) Anmeldetag : **24.07.91**

(51) Int. Cl.[5] : **C07C 235/34,** C07C 243/32, C07D 295/182, C09K 15/22, C09K 15/30, C10M 133/16, C10M 133/40, C08K 5/20, C08K 5/22, C08K 5/3435

(30) Priorität : **02.08.90 CH 2527/90**

(43) Veröffentlichungstag der Anmeldung : **11.03.92 Patentblatt 92/11**

(84) Benannte Vertragsstaaten : **DE FR GB IT**

(71) Anmelder : **CIBA-GEIGY AG**
**Klybeckstrasse 141**
**CH-4002 Basel (CH)**

(72) Erfinder : **Evans, Samuel, Dr.**
**Route des Charbonnières 17**
**CH-1723 Marly (CH)**

(54) **Orthophenolacetamide.**

(57) Es werden Zusammensetzungen, enthaltend ein thermischem, oxidativem und/oder aktinischem Abbau unterliegendes organisches Material und mindestens eine Verbindung der Formel I,

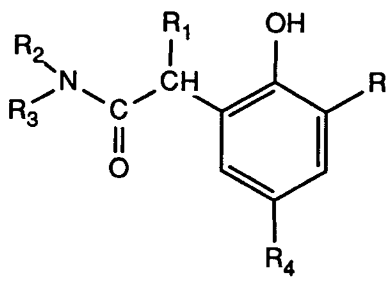

(I)

beschrieben, worin R Wasserstoff, $C_{1-24}$-Alkyl, $C_{5-12}$-Cycloalkyl, Phenyl, Phenyl-$C_{1-4}$-alkyl oder einen Rest der Formel II bedeutet, Y für -$CHR_5$-, Schwefel oder eine direkte Bindung steht, $R_1$ Wasserstoff, $C_{1-4}$-Alkyl, Phenyl oder mit Nitro, Chlor, Fluor, $C_{1-8}$-Alkyl oder/und Methoxy substituiertes Phenyl, $R_5$ Wasserstoff, $C_{1-8}$-Alkyl oder Phenyl,$R_2$ und $R_3$ unabhängig voneinander Wasserstoff, $C_{1-20}$-Alkyl, $C_{5-12}$-Cycloalkyl, Benzyl, Phenyl, mit $C_{1-18}$-Alkyl substituiertes Phenyl, $C_{2-4}$-Hydroxyalkyl, Naphthyl, Reste der Formeln $(CH_2)_x$-$COR_5$,

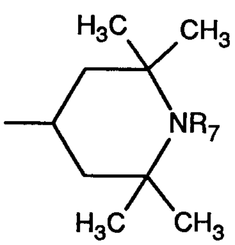

oder

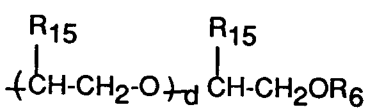

oder zusammen mit dem N-Atom, an das sie gebunden sind, einen 5-7-gliedrigen heterocyclischen Ring bedeuten, oder $R_2$ -$NHR_{14}$ ist und $R_3$ die vorstehend genannten Bedeutungen hat, $R_{14}$ Wasserstoff, $C_{1-12}$-Alkyl, Phenyl oder mit $C_{1-4}$Alkyl substituiertes Phenyl ist, $R_{15}$ Wasserstoff, $C_{1-4}$-Alkyl oder

EP 0 474 589 A1

Phenyl bedeutet und d eine ganze Zahl von 1- 18 ist, $R_6$Wasserstoff oder $C_{1-20}$-Alkyl und x eine ganze Zahl von 1 bis 5 bedeutet, $R_7$ Wasserstoff, $C_{1-8}$-Alkyl, $C_{1-12}$-Alkoxy oder CO-$R_9$ ist, $R_9$ Phenyl oder $C_{1-8}$-Alkyl ist, oder, wenn $R_3$ Wasserstoff ist und R nicht für eine Gruppe der Formel II steht, $R_2$ auch einen Rest -D-E bedeutet, wobei -D- den Formeln

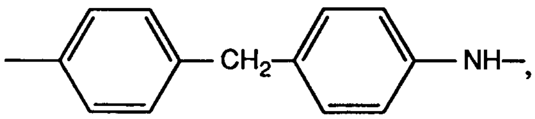 , 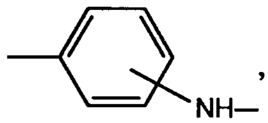 , $-C_yH_{2y}-NH-$,

$-(CH_2-CH_2-NH)_2-$ oder 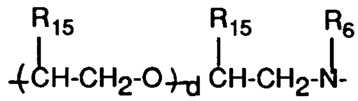

und -E den Formeln IIa

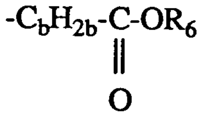

oder IIb entsprechen und die Reste E an die NH-Gruppen der Reste -D- gebunden sind, b für eine ganze Zahl von 0 bis 2 und y für eine ganze Zahl von 0 bis 12 stehen, $R_6$ Wasserstoff, $C_{1-12}$-Alkyl oder $C_{5-12}$-Cycloalkyl bedeutet, $R_{10}$ $C_{1-24}$-Alkyl oder Phenyl ist, $R_4$ Wasserstoff, $C_{1-20}$-Alkyl, $C_{5-12}$-Cycloalkyl, Benzyl, Phenyl, mit $C_{1-4}$-Alkyl substituiertes Phenyl, $C_{1-4}$-Alkoxy, $-C_nH_{2n}CO_2R_{11}$ oder III ist, $R_{11}$ Wasserstoff, $C_{1-18}$-Alkyl, $-C_zH_{2z}$-A oder $-CH_2-C(CH_2A)_3$ bedeutet, A für IIIa und z für eine ganze Zalh von 2 bis 12 stehen, n die Werte 0, 1 oder 2 annimmt, $R_{12}$ und $R_{13}$ unabhängig voneinander Wasserstoff, $C_{1-8}$-Alkyl, Phenyl oder $C_{5-12}$-Cycloalkyl bedeuten oder, wenn $R_{12}$ Methyl ist, $R_{13}$ auch für

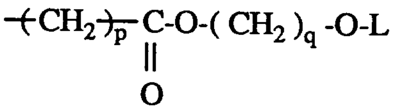

oder

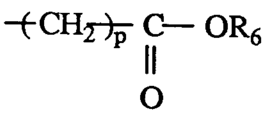

steht, L für einen Rest der Formel IIIb steht, p 1 oder 2 und q eine ganze Zahl von 2 bis 12 bedeuten, mit der Maßgabe, daß R nur einen Rest der Formel II darstellt, wenn $R_4$ nicht einer Gruppe der Formel III entspricht und das Molekül keinen Rest der Formel IIIa enthält. Die Bedeutungen der Teilformeln II, IIa, IIb, III, IIIa und IIIb sind dem Patentanspruch 1 zu entnehmen. Die Verbindungen der Formel I eignen sich insbesondere zur Stabilisierung von Schmiermitteln, Metallbearbeitungsflüssigkeiten und Hydraulikflüssigkeiten sowie von thermoplastischen Kunststoffen und Elastomeren.

Die vorliegende Erfindung betrifft Zusammensetzungen enthaltend ein thermischem, oxidativem und/oder aktinischem Abbau unterliegendes organisches Material und mindestens ein Derivat des Orthophenolacetamids sowie neue Derivate des Orthophenolacetamids und deren Verwendung zum Stabilisieren organischen Materials.

Orthophenolacetamid ist seit dem Jahre 1900 bekannt [R. Stoermer, Annalen 313, 79 ( 1900)]. Seither wurden es und seine Derivate verschiedentlich in der Naturstoff-Forschung eingesetzt [z.B. A. G. Hayes et al.,Life Sci. 34, 1241 (1984); H. Schmidhammer et al., Helv. Chim Acta 66, 2437 (1983); M. J. Calverley et al., Tetrahedron Lett. 22, 1635 (1981); K. Nagajaran et al., Proc. Indian Acad. Sci. Sect. A 86A, 25 (1977)], für organische Synthesen verwendet [z. B. A. Tsuji et al., Chem. Pharm. Bull. 20, 2528 (1972); O. Yonemitsu et al., Chem. Pharm. Bull. 19, 1158 (1971); J. Lange et al., Diss. Pharm. Pharmacol. 20, 607 (1968); J. Derkosch et al., Monatsh. 92, 542 (1961)] und für pharmazeutische Anwendungen vorgeschlagen (z. B. DE-A-1 959 898; DE-A-2 540 552; US-A-3,331,874)

Ferner ist es üblich, organische Materialien mit sogenannten "gehinderten Phenolen", d. s. Phenole mit sterisch anspruchsvollen Substituenten in den ortho-Stellungen zur OH-Gruppe, zu stabilisieren. Einschlägige Literatur ist dem Fachmann bekannt (vgl z.B. Ullmanns Enzyklopädie der technischen Chemie, 4. Auflage, Weinheim 1974, Band 8, S. 19 ff.)

Es wurde nun überraschenderweise gefunden, daß sich Derivate des Orthophenolacetamids auch sehr gut als Stabilisatoren für organische Materialien eignen.

Gegenstand der vorliegenden Erfindung sind daher Zusammensetzungen enthaltend ein thermischem, oxidativem und/oder aktinischem Abbau unterliegendes organisches Material und mindestens eine Verbindung der Formel I,

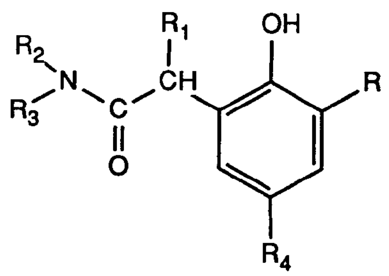

(I)

worin R Wasserstoff, $C_{1-24}$-Alkyl, $C_{5-12}$-Cycloalkyl, Phenyl, Phenyl-$C_{1-4}$-alkyl oder einen Rest der Formel II

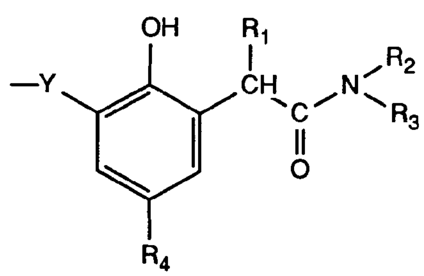

(II)

bedeutet,

Y für -$CHR_5$-, Schwefel oder eine direkte Bindung steht,

$R_1$ Wasserstoff, $C_{1-4}$-Alkyl, Phenyl oder mit Nitro, Chlor, Fluor, $C_{1-8}$-Alkyl oder/und Methoxy substituiertes Phenyl,

$R_5$ Wasserstoff, $C_{1-8}$-Alkyl oder Phenyl,

$R_2$ und $R_3$ unabhängig voneinander Wasserstoff, $C_{1-20}$-Alkyl, $C_{5-12}$-Cycloalkyl, Benzyl, Phenyl, mit $C_{1-18}$-Alkyl substituiertes Phenyl, $C_{2-4}$-Hydroxyalkyl, Naphthyl,

Reste der Formeln $(CH_2)_x$-$COR_6$,

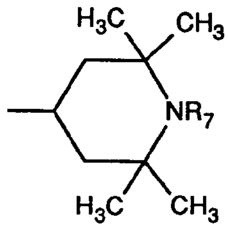 oder

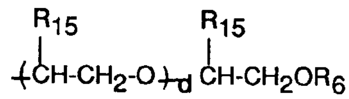

oder zusammen mit dem N-Atom, an das sie gebunden sind, einen 5-7-gliedrigen heterocyclischen Ring bedeuten,

oder $R_2$ -$NHR_{14}$ ist und $R_3$ die vorstehend genannten Bedeutungen hat,

$R_{14}$ Wasserstoff, $C_{1-12}$-Alkyl, Phenyl oder mit $C_{1-4}$Alkyl substituiertes Phenyl ist,

$R_{15}$ Wasserstoff, $C_{1-4}$-Alkyl oder Phenyl bedeutet und d eine ganze Zahl von 1- 18 ist,

$R_6$ Wasserstoff oder $C_{1-20}$-Alkyl und x eine ganze Zahl von 1 bis 5 bedeutet,

$R_7$ Wasserstoff, $C_{1-8}$-Alkyl, $C_{1-12}$-Alkoxy oder CO-$R_9$ ist,

$R_9$ Phenyl oder $C_{1-8}$-Alkyl ist,

oder, wenn $R_3$ Wasserstoff ist und R nicht für eine Gruppe der Formel II steht,

$R_2$ auch einen Rest -D-E bedeutet, wobei -D- den Formeln

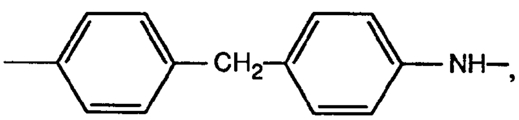, 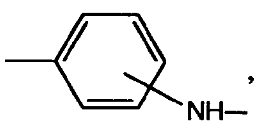, $-C_yH_{2y}-NH-$,

$-(CH_2-CH_2-NH)_2-$ oder 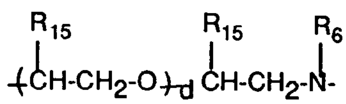 und -E den Formeln

(IIa) 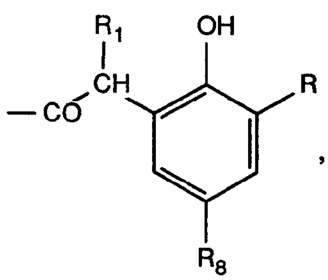, 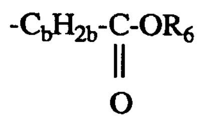 oder 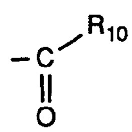 (IIb) entsprechen

und die Reste E an die NH-Gruppen der Reste -D- gebunden sind, b für eine ganze Zahl von 0 bis 2 und y für eine ganze Zahl von 0 bis 12 stehen,

$R_6$ Wasserstoff, $C_{1-12}$-Alkyl oder $C_{5-12}$-Cycloalkyl bedeutet,

$R_{10}$ $C_{1-24}$-Alkyl oder Phenyl ist,

$R_4$ Wasserstoff, $C_{1-20}$-Alkyl, $C_{5-12}$-Cycloalkyl, Benzyl, Phenyl, mit $C_{1-4}$-Alkyl substituiertes Phenyl, $C_{1-4}$-Alkoxy, -$C_nH_{2n}CO_2R_{11}$ oder

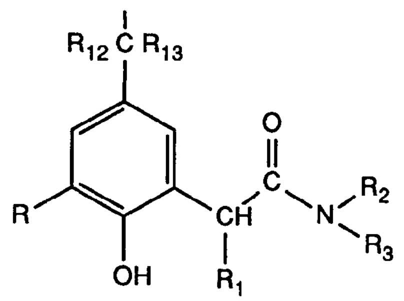

(III)

ist,

$R_{11}$ Wasserstoff, $C_{1-18}$-Alkyl, $-C_zH_{2z}$-A oder $-CH_2-C(CH_2A)_3$ bedeutet,

A für

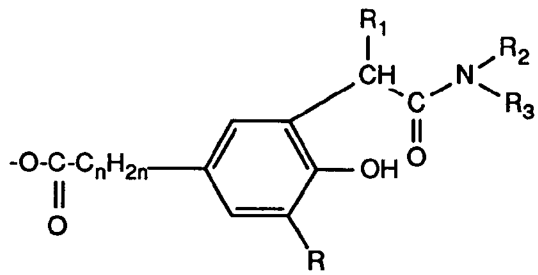

(IIIa)

und z für eine ganze Zahl von 2 bis 12 stehen, n die Werte 0, 1 oder 2 annimmt,

$R_{12}$ und $R_{13}$ unabhängig voneinander Wasserstoff, $C_{1-8}$-Alkyl, Phenyl oder $C_{5-12}$-Cycloalkyl bedeuten oder, wenn

$R_{12}$ Methyl ist, $R_{13}$ auch für

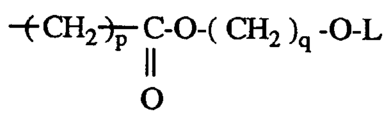

oder

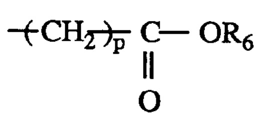

steht,

L für einen Rest der Formel

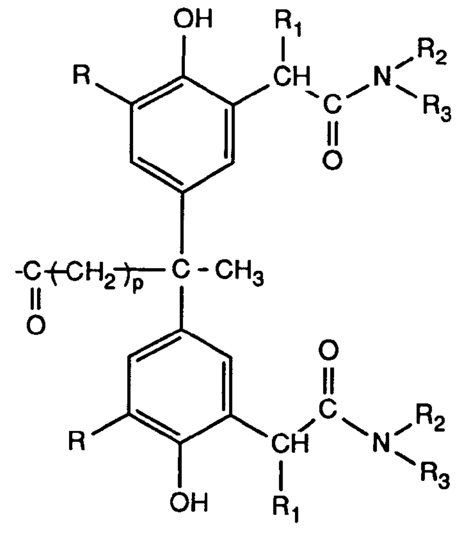

(IIIb)

steht, p 1 oder 2 und q eine ganze Zahl von 2 bis 12 bedeuten,
mit der Maßgabe daß

R nur einen Rest der Formel II darstellt, wenn $R_4$ nicht einer Gruppe der Formel III entspricht und das Molekül keinen Rest der Formel IIIa enthält.

Stellen in obigen Formeln R, $R_1$, $R_2$, $R_3$, $R_4$, $R_5$, $R_6$, $R_7$, $R_5$, $R_9$, $R_{10}$, $R_{12}$, $R_{13}$ $R_{14}$ und $R_{15}$ Alkyl dar, so handelt es sich dabei um verzweigte oder unverzweigte Reste. Der beim Symbol C im Index genannte Zahlenbereich bezieht sich dabei auf die Zahl der möglichen C-Atome. So bedeuten R und $R_{10}$ als $C_{1-24}$-Alkyl beispielsweise Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1 -Methylpentyl, 1,3-Dimethylbutyl, 1,1,3,3-Tetramethylbutyl, 1-Methylhexyl, Isoheptyl, 1 -Methylheptyl, 1,1,3-Trimethylhexyl, 1-Methylundecyl, Eicosyl, Hemicosyl oder Docosyl.

Bevorzugt sind die Alkylreste mit 1-18 C-Atomen, besonders bevorzugt für $R_{10}$ mit 1-12 C-Atomen.

$R_1$ und $R_{15}$ als $C_{1-4}$-Alkyl können z.B. Methyl, Ethyl, Propyl, Isopropyl, Butyl, Isobutyl oder tert-Butyl sein.

$R_2$ und $R_3$ als $C_{1-18}$-Alkyl können beispielhaft die für R und $R_{10}$ genannten Bedeutungen annehmen, mit Ausnahme von Eicosyl, Hemicosyl und Docosyl.

Für $R_4$ und $R_5$ als $C_{1-20}$-Alkyl sind ebenfalls die für R und $R_{10}$ genannten Beispiele anzuführen, ausgenommen Docosyl und Hemicosyl. Bevorzugt davon sind die Reste mit 1-12 C-Atomen, besonders bevorzugt die mit 1-4 C-Atomen.

$R_5$ $R_7$, $R_9$, $R_{12}$ und $R_{13}$ als $C_{1-8}$-Alkyl bedeuten beispielsweise Methyl, Ethyl, Propyl, Isopropyl, n-Butyl, Isobutyl, tert.-Butyl, Pentyl, Isopentyl, Hexyl, Heptyl, Octyl, 2-Ethylhexyl, 2-Ethylbutyl, Isoamyl, 1-Methylpentyl, 1,3-Dimethylbutyl, 1,1,3,3-Tetramethylbutyl, 1-Methylhexyl, Isoheptyl oder 1-Methylheptyl.

$R_7$, $R_{12}$ und $R_{13}$ sind bevorzugt $C_{1-4}$-Alkyl.

Beispiele für $R_5$ als $C_{1-12}$-Alkyl sind die für $R_5$, $R_7$, $R_9$, $R_{12}$ und $R_{13}$ genannten, zuzüglich Nonyl, Decyl, Undecyl, Dodecyl, 1-Methylundecyl, 2,2,4,4-Teytramethylpentyl, 1,1,3-Trimethylhexyl.

Bedeuten R, $R_1$, $R_2$, $R_3$, $R_4$, $R_5$, $R_{10}$, $R_{12}$ und $R_{13}$ $C_{5-12}$-Cycloalkyl, zählen beispielsweise dazu Cyclopentyl, Cyclohexyl, Cycloheptyl, Cyclooctyl, Cyclodecyl und Cyclododecyl. Bevorzugt sind Cyclopentyl, Cyclohexyl und Cycloheptyl, besonders bevorzugt ist Cyclohexyl.

R und $R_4$ als Phenyl-$C_{1-4}$-alkyl bedeuten z.B. Benzyl, Phenethyl, 3-Phenylpropyl, α-Methylbenzyl und α,α-Dimethylbenzyl. Bevorzugt ist Benzyl.

$R_2$ und $R_3$ als mit $C_{1-18}$-Alkyl substituiertes Phenyl können z.B. bedeuten: Methylphenyl, Dimethylphenyl, Trimethylphenyl, Ethylphenyl, Diethylphenyl, Isopropylphenyl, t-Butylphenyl, Di-t-butylphenyl, Methyl-di-t-butylphenyl, tert.-Octylphenyl und Di-tert.-octylphenyl. Die Zahl der Alkylgruppen beträgt insbesondere 1-3, z.B. 1 oder 2. Die Gesamtzahl der C-Atome aller Alkylsubstituenten ist vorzugsweise 1-18, insbesondere 1-12, z.B 1-6.

$R_2$ und $R_3$ als $C_{2-4}$-Hydroxyalkyl können beispielsweise bedeuten: 2-Hydroxyethyl, 1,2-Dihydroxyethyl, 2-Hydroxypropyl, 2,3-Dihydroxypropyl, 1,3-Dihydroxypropyl, 3-Hydroxypropyl, 4-Hydroxybutyl, sowie weitere, mit einer oder mehreren Hydroxygruppen substituierte Alkylreste mit 2-4 C-Atomen. Bevorzugt ist 2-Hydroxyethyl.

Bilden $R_2$ und $R_3$ zusammen einen 5-7-gliedrigen, heterozyklischen Ring, so kann z. B. ein solcher neben dem N-Atom noch ein N- oder O-Atom als weiteres Heteroringatom enthalten. Vorzugsweise sind die Ringe gesättigt und haben insbesondere 6 Ringglieder. Beispiele dafür sind Piperidino, Morpholino, Piperazino, 4-Methylpiperazino sowie Hexamethylenimino.

Bedeuten $R_4$ und $R_7$ $C_{1-4}$-Alkoxy, so sind Beispiele dafür Methoxy, Ethoxy, Propoxy, Isopropoxy, Butoxy und Isobutoxy.

R und $R_4$ sind bevorzugt $C_{1-4}$-Alkyl, insbesondere t-Butyl.

Bevorzugt sind erfindungsgemäße Zusammensetzungen, enthaltend mindestens eine Verbindung der Formel I, worin

R $C_{1-18}$-Alkyl, Cyclohexyl, Phenyl, Benzyl oder ein Rest der Formel II ist,

$R_1$ Wasserstoff, Methyl, Ethyl oder Phenyl darstellt,

Y für eine direkte Bindung, Methylen oder Schwefel steht,

$R_2$ und $R_3$ unabhängig voneinander Wasserstoff, $C_{1-18}$-Alkyl, Cyclohexyl, Benzyl, Phenyl, mit $C_{1-4}$-Alkyl substituertes Phenyl, Naphthyl, $-(CH_2)_xCO-R_6$, $CH_2CH_2OH$ oder einen

Rest der Formel

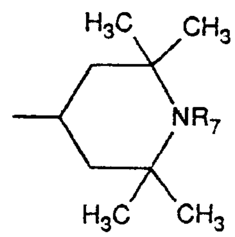

darstellen,

oder, wenn $R_2$ = H, $R_3$ auch einen Rest -D-E bedeutet, worin E eine Gruppe der Formel IIa oder IIb ist, wobei R im Rest der Formel IIa, III, IIIa und IIIb $C_{1-18}$-Alkyl Oder Cyclohexyl und $R_4$ im Rest der Formel IIa für $C_{1-12}$-Alkyl oder Cyclohexyl stehen, y 0 bis 6 bedeutet,

$R_6$ Wasserstoff oder $C_{1-4}$-Alkyl bedeutet,

$R_7$ Wasserstoff, $C_{1-4}$-Alkyl oder CO-$C_{1-4}$-Alkyl ist,

$R_4$ Wasserstoff, $C_{1-12}$-Alkyl, Cyclohexyl, Phenyl, $-C_nH_{2n}CO_2R_{11}$ oder

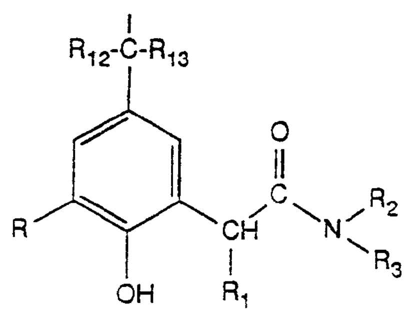

bedeutet,

$R_{12}$ und $R_{13}$ unabhängig voneinander Wasserstoff, $C_{1-4}$-Alkyl, Phenyl oder Cyclohexyl bedeuten oder, wenn $R_{12}$ Methyl ist, $R_{13}$ auch für

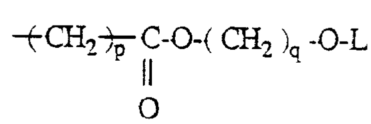

oder

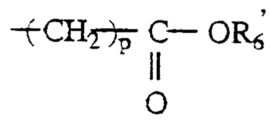

steht, $R_6'$ Wasserstoff oder $C_{1-18}$-Alkyl und q eine ganze Zahl von 2-6 darstellen.

Besonders bevorzugt sind Zusammensetzungen, enthaltend mindestens eine Verbindung der Formel I, worin
R $C_{1-18}$-Alkyl oder ein Rest der Formel II,
$R_1$ Wasserstoff oder Phenyl und
Y eine direkte Bindung ist,
$R_2$ Wasserstoff, $C_{1-20}$-Alkyl, Benzyl, Phenyl, Amino oder einen Rest der Formel

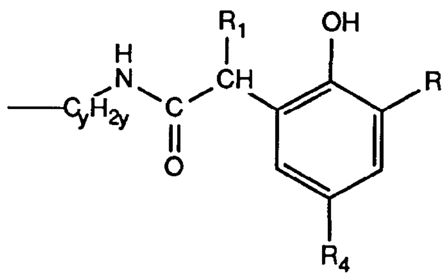

(IIIc)

oder

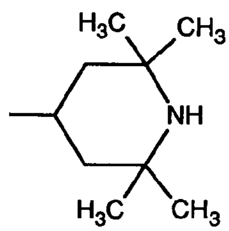

darstellt,
$R_7$ Wasserstoff, $C_{1-4}$-Alkyl oder CO-$C_{1-4}$-Alkyl ist,
$R_4$ Wasserstoff, $C_{1-6}$-Alkyl, oder eine Gruppe der Formel III bedeutet, worin $R_{12}$ und $R_{13}$ unabhängig voneinander Wasserstoff oder $C_{1-4}$-Alkyl sind, sowie, wenn $R_{12}$ Methyl ist,
$R_{13}$ auch für $-(CH_2)_2COOCH_3$ steht, $R_3$ Wasserstoff und y 2-12 bedeuten.

Ganz besonders bevorzugt sind Zusammensetzungen, enthaltend mindestens eine Verbindung der Formel I, worin
R $C_{1-6}$-Alkyl,
$R_1$ Wasserstoff oder Phenyl,
$R_2$ Wasserstoff, $C_{1-20}$-Alkyl, $NH_2$ oder einen Rest der Formel

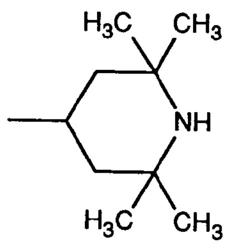

oder IIIc darstellen, worin y 2-10 ist, $R_7$ Wasserstoff, $CH_3$ oder

$-COCH_3$ ist, $R_4$ und $R_8$ Wasserstoff oder $C_{1-6}$-Alkyl bedeuten und $R_3$ für Wasserstoff steht.

Die in den erfindungsgemäßen Zusammensetzungen enthaltenen organischen Materialien können sich mehr oder weniger leicht unter Einfluß von Wärme, Licht bzw. Strahlung, mechanischer Belastung (insbesondere durch Scherkräfte) und chemischen Reagentien (besonders Luftsauerstoff) zersetzen.

Dem Schutz vor solchen Einflüssen dienen die Verbindungen der Formel I, die in den erfindungsgemäßen Zusammensetzungen zweckmäßig zu 0,01 bis 10, beispielsweise zu 0,05 bis 5, vorzugsweise zu 0,05 bis 3, insbesondere jedoch zu 0,1 bis 2 Gew.-% vorliegen sollen. Es kann sich dabei um eine oder mehrere dieser Verbindungen handeln, und die Gewichtsprozentangaben beziehen sich auf die gesamte Menge dieser Verbindungen. Berechnungsgrundlage ist dabei das Gesamtgewicht des organischen Materials ohne die Verbindungen der Formel I.

Die in den erfindungsgemäßen Zusammensetzungen enthaltenen Materialien sind solche, die gegen oxidativen, thermischen oder/und actinischen Abbau empfindlich sind. Besonders wertvoll erweisen sich dabei die Verbindungen der Formel I als Stabilisatoren gegen oxidativen und insbesondere thermischen Abbau. Sie können daher mit Vorteil als Verarbeitungs-(Thermo-)Stabilisatoren für Thermoplasten eingesetzt werden.

Ein weiterer Gegenstand der Erfindung ist daher die Verwendung von Verbindungen der Formel I zum Stabilisieren organischen Materials gegen oxidativen, thermischen und/oder actinischen Abbau sowie ein Verfahren zum Stabilisieren organischen Materials, dadurch gekennzeichnet, daß man diesem Material als Stabilisatoren Verbindungen der Formel I zusetzt bzw. auf dieses aufbringt.

Beispielhaft für organische Materialien, die erfindungsgemäß mit Hilfe der Verbindungen der Formel I stabilisiert werden können, seien erwähnt:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3.Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten- 1 -Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B Klebrigmacherharze).

4. Polystyrol, Poly-(p-methylstyrol), Poly-($\alpha$-methylstyrol).

5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkyl-acrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder a-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes

Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von $\alpha,\beta$-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit end-ständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Polym-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamid-systeme").

16. Polyharnstoffe, Polyimide, Polyamid-imide und Polybenzimidole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wäßrige Emulsionen.

29. Wäßrige Emulsionen natürlicher oder synthetischer Kautschuke, wie z.B. Naturkautschuk-Latex oder Latices von carboxylierten Styrol-Butadien-Copolymeren.

Die erfindungsgemäßen Zusammensetzungen enthalten als organisches Material vorzugsweise natürliche, halbsynthetische oder synthetische Polymere, einen Schmierstoff, eine Metallbearbeitungsflüssigkeit oder eine Hydraulikflüssigkeit. Bevorzugt sind Zusammensetzungen, die ein synthetisches Polymer enthalten, insbesondere einen thermoplastischen Kunststoff oder ein Elastomer. Vor allem sind Zusammensetzungen hervorzuheben, die ein Polyolefin als organisches Material enthalten. Beispiele für solche Polymere sind der vorstehenden Aufzählung von geeigneten Materialien zu entnehmen.

Ebenfalls besonders bevorzugt sind Zusammensetzungen, die einen Schmierstoff, eine Metallbearbeitungsflüssigkeit oder eine Hydraulikflüssigkeit, insbesondere einen Schmierstoff, enthalten.

Die in Frage kommenden Schmierstoffe basieren beispielsweise auf mineralischen oder synthetischen Ölen oder Mischungen davon. Die Schmierstoffe sind dem Fachmann geläufig und in der einschlägigen Fachliteratur, wie beispielsweise in Dieter Klamann, "Schmierstoffe und verwandte Produkte" (Verlag Chemie, Weinheim, 1982), in Schewe-Kobek, "Das Schmiermittel-Taschenbuch" (Dr. Alfred Hüthig-Verlag, Heidelberg, 1974) und in "Ullmanns Enzyklopädie der technischen Chemie", Bd. 13, Seiten 85-94 (Verlag Chemie, Weinheim, 1977) beschrieben.

Die Schmierstoffe sind insbesondere Öle und Fette, beispielsweise basierend auf einem Mineralöl. Bevorzugt sind Öle.

Eine weitere Gruppe von Schmierstoffen, die zur Anwendung gelangen können, sind pflanzliche oder tierische Öle, Fette, Talge und Wachse oder deren Gemische untereinander oder Gemische mit den erwähnten mineralischen oder synthetischen Oelen. Pflanzliche und tierische Öle, Fette, Talge und Wachse sind beispielsweise Palmkernöl, Palmöl, Olivenöl, Rueböl, Rapsöl, Leinöl, Erdnußöl, Sojabohnenöl, Baumwollöl, Sonnenblumenöl, Kürbiskernöl, Kokosnußöl, Maisöl, Rizinusöl, Baumnußöl und Mischungen davon, Fischöle, Talge von Schlachttieren wie Rindertalg, Klauenfett und Knochenöl sowie deren modifizierte, epoxidierte und sulfoxidierte Formen, beispielsweise epoxidiertes Sojabohnenöl.

Die Mineralöle basieren insbesondere auf Kohlenwasserstoffverbindungen.

Beispiele von synthetischen Schmierstoffen umfassen Schmierstoffe auf der Basis der aliphatischen oder aromatischen Carboxylester, der polymeren Ester, der Polyalkylen-oxide, der Phosphorsäureester, der Poly-$\alpha$-olefine oder der Silicone, eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropan-tricaprylat oder Gemische davon, eines Tetraesters von Pentaerythrit mit einer einwertigen Säure oder mit einem Gemisch solcher Säuren, wie z.B. Pentaerythrit-tetracaprylat, oder eines komplexen Esters von einwertigen und zweiwertigen Säuren mit mehrwertigen Alkoholen, z.B. ein komplexer Ester von Trimethylolpropan mit Capryl-und Sebacinsäure oder von einem Gemisch davon. Besonders geeignet sind neben Mineralölen z.B. Poly-$\alpha$-Olefine, Schmierstoffe auf Esterbasis, Phosphate, Glykole, Polyglykole und Polyalkylenglykole, sowie deren Mischungen mit Wasser.

Metallbearbeitungsflüssigkeiten und Hydraulikflüssigkeiten können auf Basis der gleichen Substanzen hergestellt werden wie vorstehend für die Schmiermittel beschrieben. Häufig handelt es sich dabei um Emulsionen solcher Substanzen in Wasser oder anderen Flüssigkeiten.

Die Einarbeitung in die organischen Materialien kann beispielsweise durch Einmischen der Verbindungen der Formel I und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindungen auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der Verbindungen der Formel I in Polymere besteht in deren Zugabe vor, während oder unmittelbar nach der Polymerisation der entsprechenden Monomeren bzw. vor der Vernetzung. Die Verbindungen der Formel I können dabei als solche, aber auch in encapsulierter Form (z.B in Wachsen, Ölen oder Polymeren) zugesetzt werden. Im Falle der Zugabe vor oder während der Polymerisation können die Verbindungen der Formel I auch als Regulatoren für die Kettenlänge der Polymeren (Kettenabbrecher) wirken.

Die Verbindungen der Formel I oder Mischungen davon können auch in Form eines Masterbatches, der diese Verbindungen beispielsweise in einer Konzentration von 2,5 bis 25 Gew.-% enthält, den zu stabilisierenden Kunststoffen zugesetzt werden.

Zweckmäßig kann die Einarbeitung der Verbindungen der Formel I nach folgenden Methoden erfolgen:

- als Emulsion oder Dispersion (z.B. zu Latices oder Emulsionspolymeren)
- Als Trockenmischung während des Vermischens von Zusatzkomponenten oder Polymermischungen
- durch direktes Zugeben in die Verarbeitungsapparatur (z.B. Extruder, Innenmischer usw.)
- als Lösung oder Schmelze.

Erfindungsgemäße Polymerzusammensetzungen können in verschiedener Form angewendet bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Erfindungsgemäße Schmierstoffzusammensetzungen finden z.B. Verwendung in Verbrennungsmotoren, z.B. in Kraftfahrzeugen.

Zusätzlich zu den erfindungsgemäßen Verbindungen bzw. Mischungen können die erfindungsgemäßen Zusammensetzungen, insbesondere wenn sie organische, vorzugsweise synthetische, Polymere enthalten, noch weitere übliche Additive enthalten. Beispiele für solche Additive sind:

1. Antioxidantien

1. 1. Alkylierte Monophenole, z.B. 2,6-Di-tert.butyl-4-methylphenol, 2-Tert.butyl-4,6-di-methylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tricyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2,2′-Thio-bis-(6-tert.-butyl-4-methylphenol), 2,2′-Thio-bis-(4-octylphenol), 4,4′-Thio-bis-(6-tert.butyl-3-methylphenol), 4,4′-Thio-bis-(6-tert.butyl-2-methylphenol).

1.4. Alkyliden-Bisphenole, z.B. 2,2′-Methylen-bis-(6-tert.butyl-4-methylphenol), 2,2′-Methylen-bis-(6-tert.butyl-4-ethylphenol), 2,2′-Methylen-bis-[4-methyl-6-(α-methyl-cyclohexyl)-phenol], 2,2′-Methylen-bis-(4-methyl-6-cyclohexylphenol), 2,2′-Methylen-bis-(6-nonyl-4-methylphenol), 2,2′-Methylen-bis-(4,6-di-tert.butylphenol), 2,2′Ethyliden-bis-(4,6-di-tert.butylphenol), 2,2′-Ethyliden-bis-(6-tert.butyl-4-isobutylphenol), 2,2′-Methylen-bis-[6-(α-methylbenzyl)-4-nonylphenol], 2,2′-Methylen-bis-[6-(α,α-di-methylbenzyl)-4-nonylphenol], 4,4′-Methylen-bis-(2,6-di-tert.-butylphenol), 4,4′-Methylen-bis- (6-tert.butyl-2-methylphenol), 1,1-Bis-(5-tert.butyl-4-hydroxy-2-methyl-phenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methyl-phenyl)-3-n-dodecylmercaptobutan, Ethylenglycol-bis-[3,3-bis-(3′-tert.-butyl-4′-hydroxyphenyl)-butyrat], Bis-(3-tert.butyl-4-hydroxy-5-methyl-phenyl)-dicyclopentadien, Bis-[2-(3′-tert.butyl-2′-hydroxy-5′-methyl-benzyl)-6-tert.butyl-4-methyl-phenyl]-terephthalat.

1.5 Benzylverbindungen, z.B. 1,3,5-Tris-(3,5-di-tert.butyl-4-hydroxy-benzyl)-2,4,6-trimethylbenzol, Bis-(3,5-di-tert.butyl-4-hydroxybenzyl)-sulfid, 3,5-Di-tert.buty-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino )-s-triazin, N-(3,5-di-tert.-butyl-4-hydroxyphenyl)-carbaminsäureoctylester.

1.7. Ester der ß-(3,5-Di-tert.butyl-4-hydroxyphenyl)-propionsäure mit ein- oder mehrwertigen Alkoholen, wie z.B. mit Methanol, Octa- decanol, 1,6-Hexandiol, Neopentylglycol, Thiodiethylenglycol, Diethylenglycol, Triethylenglycol, Pentaerythrit, Tris(hydroxyethyl)-isocyanurat, N,N′-Bis-(hydroxyethyl)-oxalsäurediamid.

1.8. Ester der ß-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.9. Ester der ß-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.10 Amide der ß-(3,5-Di-tert.butyl-4-hydroxyphenyl)-propionsäure, wie z.B. N,N′-Bis-(3,5-di-tert.butyl-4-hydroxyphenylpropionyl)-hexamethylendiamin, N,N′-Bis-(3,5-di-tert.-butyl-4-hydroxyphenylpropionyl)-trimethylendiamin, N,N′-Bis-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl-,3',5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyl-oxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenyl-salicylat, Phenylsalicylat, Octylphenyl-salicylat, Dibenzoylresorcin, Bis-(4-tert.butyl-benzoyl)-resorcin, Benzoylresorcin, 3,5-Di-tert.butyl-4-hydroxybenzoesäure-2,4-di-tert.-butylphenylester,
3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethyl-ester bzw. -butylester, α-Carbomethoxy-p-methoxy-zimtsäure-methylester, N-(β-Carbo-methoxy-β-cyanovinyl)-2-methyl-indolin.

2.5. Nickelverbindungen, wie z.B. Nickelkomplexe des 2,2'-Thio-bis-[4-(1,1,3,3-tetra-methylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzyl-phosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethy-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.-butyl-oxanilid, 2,2'-Di-dodecyloxy-5,5'di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)- 1,3,5-triazin, 2-(2-Hydroxy-4-octyloxyphenyl)-4,6-bis(4-methylphenyl)-1,3,5-triazin, 2-(2-Hydroxy-4-dodecyloxy-phenyl)-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxy-phenylpropionyl)-hydrazin, 3-Salicyloylamino-1,2,4-triazol, Bis-(benzyliden)-oxalsäure-dihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tri-stearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.

10. Sonstige Zusätze, wie z.B. Weichmacher, Gleitmittel, Emulgatoren, Pigmente, Optische Aufheller, Flammschutzmittel, Antistatika, Treibmittel.

Handelt es sich bei den erfindungsgemäßen Zusammensetzungen um solche auf Basis von Schmierstoffen und Hydraulikflüssigkeiten bzw. Metallbearbeitungsflüssigkeiten, können diese ebenfalls weitere Additive enthalten, die zugegeben werden, um gewisse Gebrauchseigenschaften zu verbessern, wie z.B. weitere Antioxidantien, Metalldesaktivatoren, Rostinhibitoren, Viskositäts-Index-Verbesserer, Stockpunkterniedriger, Dispergiermittel/Tenside und Verschleißschutzadditive.

Beispiele für Antioxidantien sind der weiter oben wiedergegebenen Auflistung unter dem Titel "1. Antioxidantien", insbesondere Punkte 1.1 bis 1.10 zu entnehmen. Beispiele für weitere zusätzliche Additive sind die folgenden:

Beispiele für aminische Antioxidantien:

N,N′-Di-isopropyl-p-phenylendiamin, N,N′-Di-sec-butyl-p-phenylendiamin, N,N′-Bis-(1,4-dimethyl-pentyl)-p-phenylendiamin, N,N′-Bis(1-ethyl-3-methyl-pentyl)-p-phenylen-diamin, N,N′-Bis(1-methyl-heptyl)-p-phenylendiamin, N,N′-Dicyclohexyl-p-phenylendiamin, N,N′-Diphenyl-p-phenylendiamin, N,N′-Di-(naphthyl-2)-p-phenylendiamin, N-Isopropyl-N′-phenyl-p-phenylendiamin, N-(1,3-Dimethyl-butyl)-N′-phenyl-p-phenylendiamin, N-(1-Methyl-heptyl)-N′-phenyl-p-phenylendiamin, N-Cyclohexyl-N′phenyl-p-phenylendiamin, 4-(p-Toluolsulfonamido)-diphenylamin, N,N′-Dimethyl-N,N′-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p′-Di-tert-octyldiphenylamin, 4-n-Butylaminophenol, 4-Butyryl-amino-phenol, 4-Nonanoylamino-phenol, 4-Dodecanoylamino-phenol, 4-Octadecanoyl-amino-phenol, Di-(4-methoxyphenyl)-amin, 2,6-Di-tert-butyl-4-dimethylamino-methyl-phenol, 2,4′-Diamino-diphenylmethan, 4,4′-Diamino-diphenylmethan, N,N,N′,N′-Tetramethyl-4,4′-diamino-diphenylmethan, 1,2-Di-[(2-methyl-phenyl)-amino]-ethan, 1,2-Di-(phenylamino)-propan, (o-Tolyl)-biguanid, Di-[4-(1′,3′-dimethyl-butyl)-phenyl]amin,tert-octyliertes N-Phenyl-1-naphthylamin, Gemisch aus mono- und dialkylierten tert-Butyl/tert-Octyldiphenylaminen, 2,3-Dihydro-3,3-dimethyl-4H-1,4-benzothiazin, Phenothiazin, N-Allylphenothiazin.

Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure.

Beispiele für Metall-Desaktivatoren, z.B. für Kupfer, sind:

Triazole, Benztriazole und deren Derivate, Tolutriazole und deren Derivate, 2-Mercaptobenzthiazol, 2-Mercaptobenztriazol, 2,5-Dimercaptobenztriazol, 2,5-Dimercaptobenzthiadiazol, 5,5′-Methylenbisbenztriazol, 4,5,6,7-Teaahydrobenztriazol, Salicyliden-propylendiamin, Salicylaminoguanidin und dessen Salze.

Beispiele für Rost-Inhibitoren sind:

a) Organische Säuren, ihre Ester, Metallsalze und Anhydride, z.B.:
N-Oleoyl-sarcosin, Sorbitan-mono-oleat, Blei-naphthenat, Alkenylbernsteinsäure-anhydrid, z.B. Dodecenylbernsteinsäure-anhydrid, Alkenylbernsteinsäure-Teilester und -Teilamide, 4-Nonylphenoxy-essigsäure.

b) Stickstoffhaltige Verbindungen, z.B.:
- I. Primäre, sekundäre oder tertiäre aliphatische oder cycloaliphatische Amine und Amin-Salze von organischen und anorganischen Säuren, z.B. öllösliche Alkyl-ammoniumcarboxylate.
- II. Heterocyclische Verbindungen, z.B.:
Substituierte Imidazoline und Oxazoline.

c) Phosphorhaltige Verbindungen, z.B.:
Aminsalze von Phosphorsäurepartialestern oder Phosphonsäurepartialestern, Zinkdialkyldithiophosphate.

d) Schwefelhaltige Verbindungen, z.B.:
Barium-dinonylnaphthalin-sulfonate, Calciumpetroleum-sulfonate.

Beispiele für Viskositätsindex-Verbesserer sind:

Polyacrylate, Polymethacrylate, Vinylpyrrolidon/Methacrylat-Copolymere, Polyvinyl-pyrrolidone, Polybutene, Olefin-Copolymere, Styrol/Acrylat-Copolymere, Polyether.

Beispiele für Stockpunkterniedriger sind:

Polymethacrylat, alkylierte Naphthalinderivate.

Beispiele für Dispergiermittel/Tenside sind:

Polybutenylbernsteinsäureamide oder -imide, Polybutenylphosphonsäurederivate, basische Magnesium-, Calcium-, und Bariumsulfonate und -phenolate.

Beispiele für Verschleißschutz-Additive sind:

Schwefel und/oder Phosphor und/oder Halogen enthaltende Verbindungen, wie geschwefelte pflanzliche Oele, Zinkdialkyldithiophosphate, Tritolylphosphat, chlorierte Paraffine, Alkyl- und Aryldi- und tri-sulfide, Triphenylphosphorothionate, Diethanol-aminomethyltolyltriazol, Di(2-ethylhexyl)aminomethyltolyltriazol.

Ein weiterer Geganstand der Erfindung sind neue Verbindungen der Formel I, wie sie weiter oben definiert ist, mit der zusätzlichen Maßgbe, daß $R_2$ nitcht Wasserstoff, $C_{3-12}$-Alkyl, Phenyl, Cyclopentyl oder Acetyl bedeutet, wenn $R_3$ Wasserstoff ist und gleichzeitig R für Wasserstoff oder Methyl steht.

Bevorzugte neue Verbindungsgruppen entsprechen den oben in Zusammenhang mit den erfindungsgemäßen Zusammensetzungen erwähnten bevorzugten Gruppen von Verbindungen der Formel I. Das gleiche gilt für allgemeine und spezifische Substituentenbedeutungen.

Soweit die Verbindungen der Formel I nicht bekannt sind (siehe die eingangs genannten Literaturstellen), können sie in Analogie zu an sich bekannten Verfahren hergestellt werden. Dies wird im folgenden beispielhaft näher erläutert.

Herstellung der Verbindungen der Formel I

1. Ausgangsverbindungen

Die benötigten Ausgangsverbindungen werden z. B. nach den in WO -A-80/01566, S. 25, US-A-3,862,133 sowie in R. LAMER, J. HET. CHEM. 12, 1067 (1975) beschriebenen Verfahren hergestellt.

Schematisch läßt sich die Herstellung folgendermaßen darstellen:

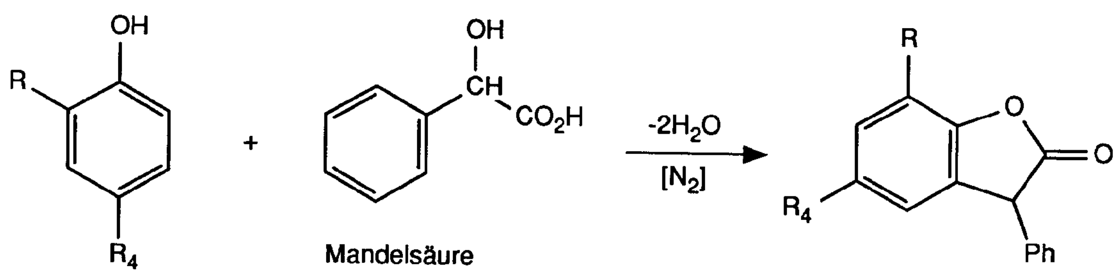


Mandelsäure

bzw.

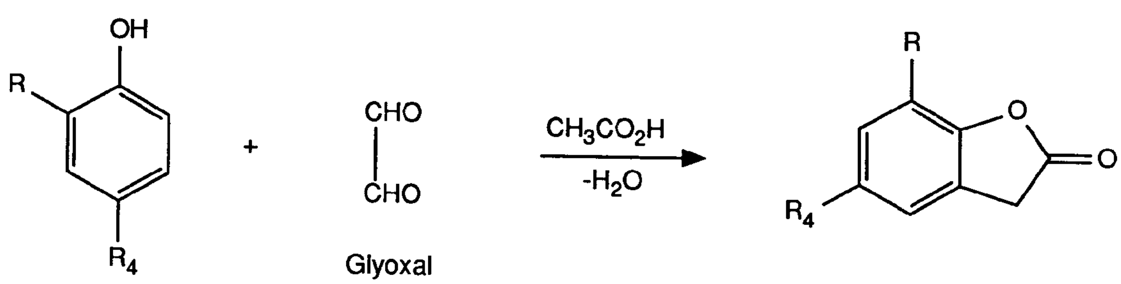


Glyoxal

Mandelsäure und Glyoxal sind kommerziell erhältlich, und das substituierte Phenol wird nach an sich bekannten Verfahren der organischen Chemie hergestellt, soweit es nicht selbst kommerziell verfügbar ist. Verwendet man die am Phenylring entsprechend substituierte Mandelsäure bzw. alkylsubstituiertes Glyoxal,

kommt man zu den Ausgangsprodukten, die den zusätzlichen Bedeutungsmöglichkeiten von $R_1$ Rechnung tragen.

2. Die Amidierung unter Ringöffnung für zu den erfindungsgemäßen Verbindungen:

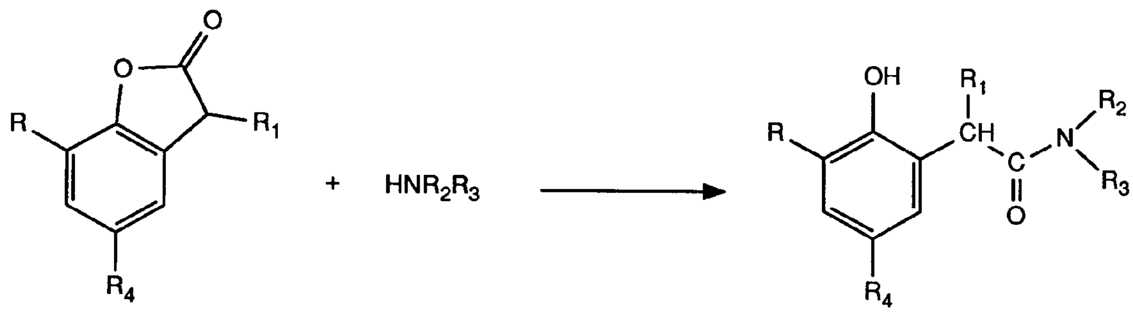


bzw.

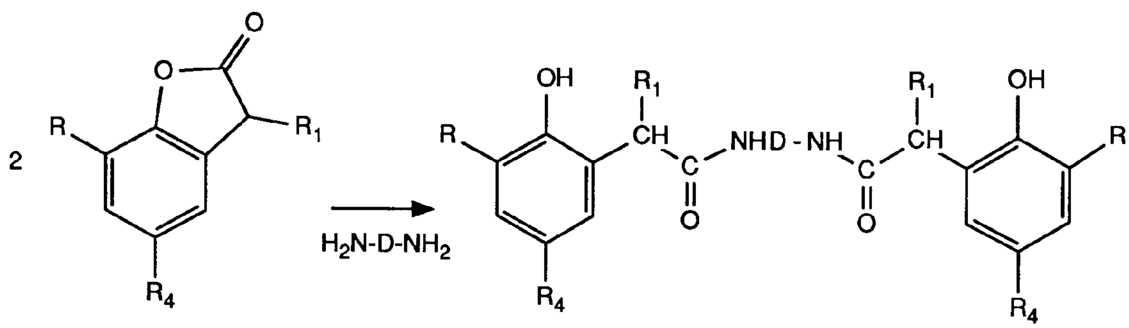


Die Reaktion kann bei 70 bis 200°C durchgeführt werden, bevorzugt bei 110 bis 150°C, und zwar mit oder ohne Lösungsmittel. Werden Lösungsmittel verwendet (insbesondere bei Vorhandensein langkettiger Alkylreste), so sind hochsiedende organische Lösungsmittel geeignet, wie z.B. Ligroinfraktionen, Benzol, Toluol, Xylol, weitere alkylierte sowie auch chlorierte Benzolabkömmlinge, DMF, DMA, DMSO, genügend hoch siedende Alkohole wie Ethanol, Propanol, Butanol und Hexanol. Für den Fall, daß$R_3$ Wasserstoff und $R_2$ -$NHR_{14}$ ist, wird die Amidierung direkt mit Hydrazin oder dessen Derivaten durchgeführt. In diesem Fall kann die Reaktionstemperatur zwischen 5 und 150°C, bevorzugt zwischen 20 und 40°C liegen. Außer den vorstehend genannten Lösungsmitteln können dann auch THF und Dioxan verwendet werden. Nähere Details sind auch den nachfolgenden Herstellungsbeispielen zu entnehmen.

Die folgenden Beispiele erläutern die Erfindung weiter, ohne sie jedoch zu beschränken. Soweit nichts anderes angegeben ist, bedeuten darin sowie in der übrigen Beschreibung Teile- und Prozentangaben Gewichtsteile und Gewichtsprozent. t-Bu bedeutet den Rest $-C(CH_3)_3$.

Beispiel 1

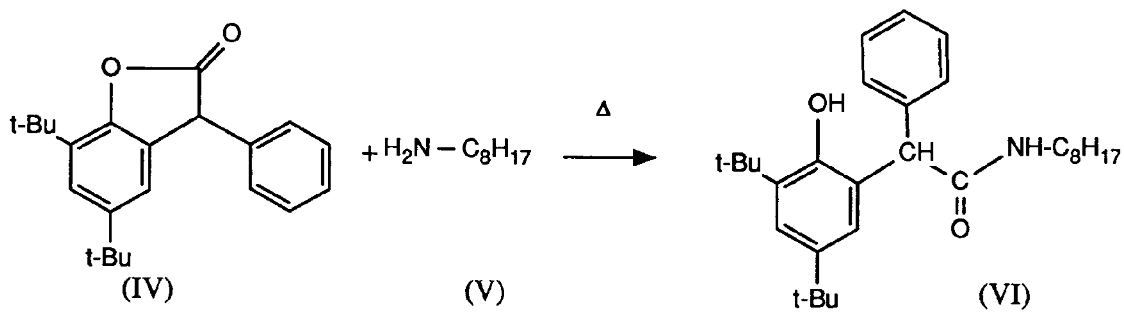


(IV) (V) (VI)

In einem 750 ml Sulfierkolben mit Thermometer, Kühler, $N_2$-Überleitung werden 161.2 g (0.5 mol) der Verbindung IV vorgelegt und 64.6 g 1-Octylamin (V) zugegeben. Es wird auf 125°C erhitzt und 3 h bei dieser Temperatur belassen. Nach Lösen in 800 ml heißem Isopropanol/Wasser (85/15) wird im Eisbad abgekühlt und der entstandene weiße Rückstand abgesaugt. Nach Waschen mit 200 ml Isopropanol/Wasser (85/15) und Trocknen bei 60°C erhält man 213.4 g (94.5% d.Th.) eines weißen Pulvers (Verbindung VI). Schmelzpunkt 118°C.

| Analyse: | berechnet: | | [%] | gefunden: [%] | | |
|---|---|---|---|---|---|---|
| | C | H | N | C | H | N |
| | 79.77 | 10.04 | 3.10 | 80.04 | 9.89 | 3.15 |

Die Verbindung der Formel IV wird gemäß WO-A-80/01566, S.25, erhalten.

Beispiel 2

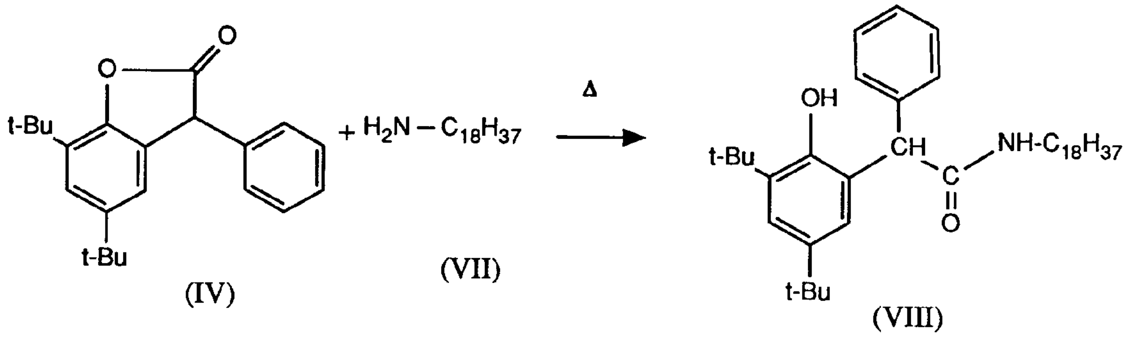

(IV) (VII) (VIII)

32.2 g (0.1 mol) der Verbindung IV werden analog Beispiel 1 mit 26.9 g (0.1 mol) 1-Octadecylamin (VII) umgesetzt, das erhaltene Produkt aus 150 ml Methanol umkristallisiert und bei 60°C getrocknet. Man erhält 49 g (82.8 % d. Th.) eines weißen Pulvers (Verbindung VIII). Schmelzpunkt 98°C.

| Analyse: | berechnet: | | [%] | gefunden: [%] | | |
|---|---|---|---|---|---|---|
| | C | H | N | C | H | N |
| | 81.16 | 11.07 | 2.37 | 81.14 | 11.06 | 2.27 |

Beispiel 3

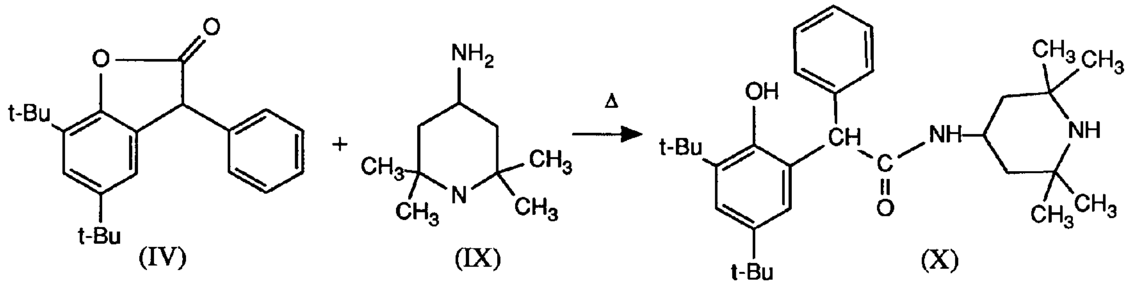

(IV) (IX) (X)

16.1 g (0.05 mol) der Verbindung IV werden mit 7.8 g (0.05 mol) 4-Amino-2.2.6.6-tetramethylpiperidin(IX) analog Beispiel 1 bei 140°C während 8 h umgesetzt. Das Produkt wird in n-Hexan suspendiert, abgesaugt und bei 60°C getrocknet. Man erhält 22.8 g (95.3% d. Th.) eines weißen Pulvers (Verbindung X). Schmelzpunkt: 199°C.

| Analyse: | berechnet: | | [%] | gefunden: [%] | | |
|---|---|---|---|---|---|---|
| | C | H | N | C | H | N |
| | 77.78 | 9.69 | 5.85 | 77.95 | 9.75 | 5.74 |

Beispiel 4

64.5 g (0.2 mol) der Verbindung IV werden mit 11.6 g (0.1 mol) 1.6-Diaminohexan (XI) analog Beispiel 3 umgesetzt und aufgearbeitet. Man erhält 58.3 g (76.6% d. Th.) eines weißen Pulvers (Verbindung XII) mit einem Schmelzpunkt von 195°C.

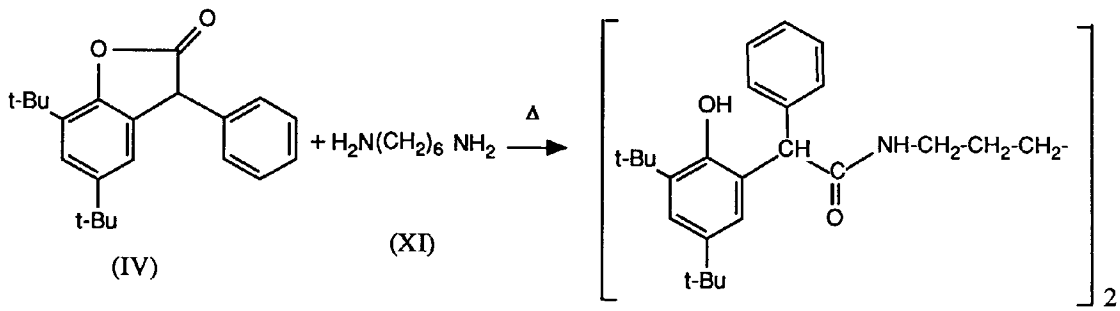


(IV) (XI) (XII)

| Analyse: | berechnet: | | [%] | gefunden: [%] | | |
|---|---|---|---|---|---|---|
| | C | H | N | C | H | N |
| | 78.91 | 9.01 | 3.68 | 78.83 | 8.89 | 3.65 |

Beispiel 5

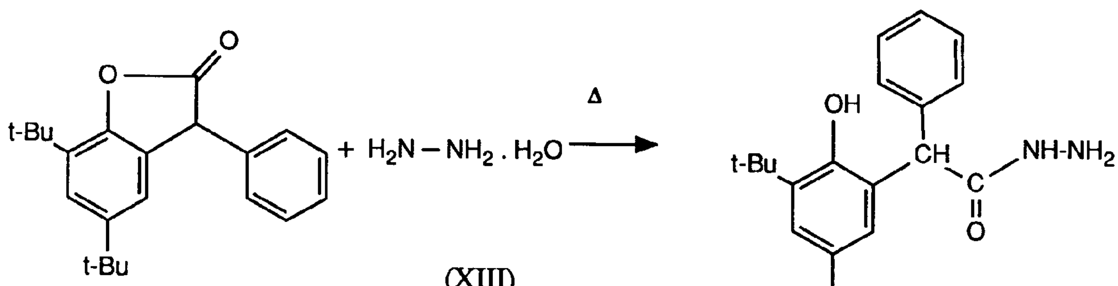


(IV) (XIII) (XIV)

In einem 750 ml Sulfierkolben mit Thermometer, Rückflusskühler, Stickstoffüberleitung und Tropftrichter werden 64.5 g (0.2 mol) der Verbindung IV in 300 ml Ethanol vorgelegt. Zu dieser klaren, farblosen Lösung wird während 30 min 10 g (0.2 mol) Hydrazinhydrat (XIII) zugetropft. Es entsteht ein Kristallbrei, der mit 250 ml Wasser verdünnt und abgesaugt wird. Nach Waschen mit 200 ml Ethanol/Wasser (1:1) wird das erhaltene, weisse Pulver (Verbindung XIV) bei 60°C getrocknet.
Ausbeute: 68 g (95.9% d.Th.), Schmelzpunkt 146°C.

| Analyse: | berechnet: | | [%] | gefunden: [%] | | |
|---|---|---|---|---|---|---|
| | C | H | N | C | H | N |
| | 74.54 | 8.53 | 7.90 | 74.59 | 8.55 | 7.77 |

Beispiel 6: Test auf Stabilisierung von Polypropylen bei Mehrfachextrusion

1.3 kg Polypropylenpulver (Schmelzindex 3.2g/10 min., gemessen bei 230°C mit 2.16 kg) werden mit 0.05% Calziumstearat, 0.05% Irganox® 1010 (Ciba-Geigy) und 0.05% der Verbindung aus Beispiel 1,3 oder 5 vermischt. Diese Mischung wird in einem Extruder mit einem Zylinderdurchmesser von 20 mm und einer Länge von 400 mm mit 100 U/min extrudiert, wobei die drei Heizzonen auf die folgenden Temperaturen eingestellt werden: 260°C, 270°C und 280°C. Das Extrudat wird zur Kühlung durch ein Wasserbad gezogen und anschließend granuliert. Nach zwei weiteren, in gleicher Weise verlaufenden Extrusionen wird der Schmelzindex gemessen (bei 230°C mit 2.16 kg). Große Zunahme des Schmelzindex bedeutet starken Kettenabbau, also schlechte Stabilisierung.

Die Ergebnisse sind in der folgenden Tabelle zusammengefaßt:

| Verbindung aus Beispiel | Schmelzindex |
|---|---|
| 1 | 4.6 |
| 3 | 4.6 |
| 5 | 4.8 |
| ohne Additiv | 16.5 |

**Patentansprüche**

1. Zusammensetzung enthaltend ein thermischem, oxidativem und/oder aktinischem Abbau unterliegendes organisches Material und mindestens eine Verbindung der Formel I,

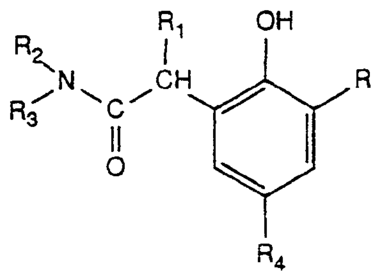

(I)

worin R Wasserstof, $C_{1-24}$-Alkyl, $C_{5-12}$-Cycloalkyl, Phenyl, Phenyl-$C_{1-4}$-alkyl oder einen Rest der Formel II

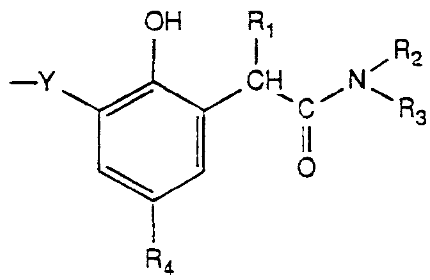

(II)

bedeutet,

Y für $-CHR_5-$, Schwefel oder eine direkte Bindung steht,

$R_1$ Wasserstoff, $C_{1-4}$-Alkyl, Phenyl oder mit Nitro, Chlor, Fluor, $C_{1-8}$-Alkyl oder/und Methoxy substituiertes Phenyl,

$R_5$ Wasserstoff, $C_{1-8}$-Alkyl oder Phenyl,

$R_2$ und $R_3$ unabhängig voneinander Wasserstoff, $C_{1-20}$-Alkyl, $C_{5-12}$-Cycloakyl,

Benzyl, Phenyl, mit $C_{1-18}$-Alkyl substituiertes Phenyl, $C_{2-4}$-Hydroxyalkyl, Naphthyl,

Reste der Formeln $(CH_2)_x-COR_6$,

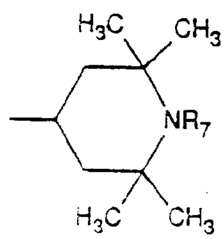

oder

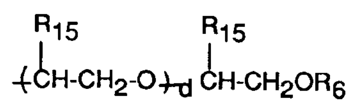

oder zusammen mit dem N-Atom, an das sie gebunden sind, einen 5-7-gliedrigen heterocyclischen Ring bedeuten,

oder $R_2$ $-NHR_{14}$ ist und $R_3$ die vorstehend genannten Bedeutungen hat,

$R_{14}$ Wasserstoff, $C_{1-12}$-Alkyl, Phenyl oder mit $C_{1-4}$Alkyl substituiertes Phenyl ist,

$R_{15}$ Wasserstoff, $C_{1-4}$-Alkyl oder Phenyl bedeutet und d eine ganze Zahl von 1- 18 und x eine ganze Zahl von 1 bis 5 ist,

$R_6$ Wasserstoff oder $C_{1-20}$-Alkyl bedeutet,

$R_7$ Wasserstoff, $C_{1-8}$-Alkyl, $C_{1-12}$-Alkoxy oder $CO-R_9$ ist,

$R_9$ Phenyl oder $C_{1-8}$-Alkyl ist,

oder, wenn $R_3$ Wasserstoff ist und R nicht für eine Gruppe der Formel II steht,

$R_2$ auch einen Rest -D-E bedeutet, wobei -D- den Formeln

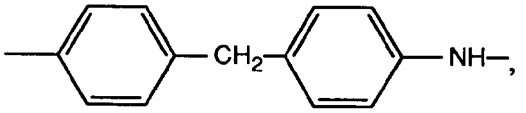, 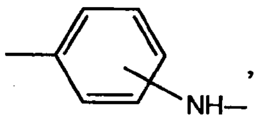, $-C_yH_{2y}-NH-$,

$-(CH_2-CH_2-NH)_2-$ oder 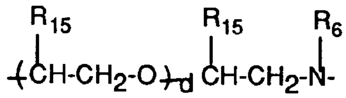 und -E den Formeln

(IIa) 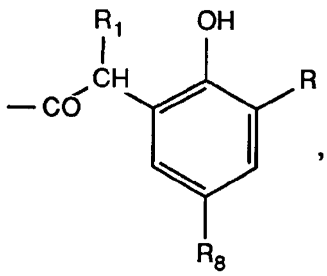, 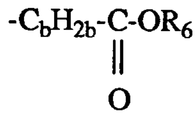 oder 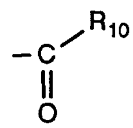 (IIb) entsprechen

und die Reste E an die NH-Gruppen der Reste -D- gebunden sind, b für eine ganze Zahl von 0 bis 2 und y für eine ganze Zahl von 0 bis 12 stehen,

$R_8$ Wasserstoff, $C_{1-12}$-Alkyl oder $C_{5-12}$-Cycloalkyl bedeutet,

$R_{10}$ $C_{1-24}$-Alkyl oder Phenyl ist,

$R_4$ Wasserstoff, $C_{1-20}$-Alkyl, $C_{5-12}$-Cycloalkyl, Benzyl, Phenyl, mit $C_{1-4}$-Alkyl substituiertes Phenyl, $C_{1-4}$-Alkoxy, $-C_nH_{2n}CO_2R_{11}$ oder

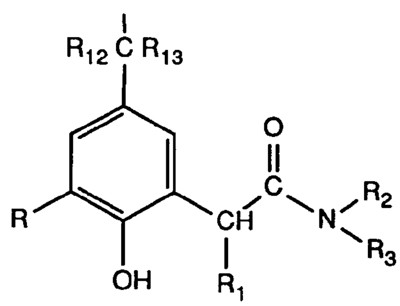

(III)

ist,

$R_{11}$ Wasserstoff, $C_{1-18}$-Alkyl, $-C_zH_{2z}-A$ oder $-CH_2-C(CH_2A)_3$ bedeutet,

A für

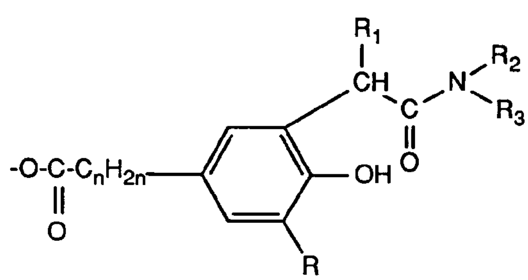

(IIIa)

und z für eine ganze Zahl von 2 bis 12 stehen, n die Werte 0, 1 oder 2 annimmt,

$R_{12}$ und $R_{13}$ unabhängig voneinander Wasserstoff, $C_{1-18}$-Alkyl, Phenyl oder $C_{5-12}$-Cycloalkyl bedeuten

oder, wenn

$R_{12}$ Methyl ist, $R_{13}$ auch für

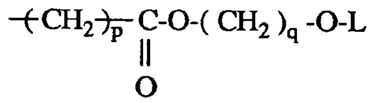

oder

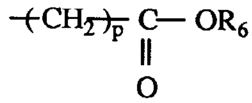

steht,

L für einen Rest der Formel

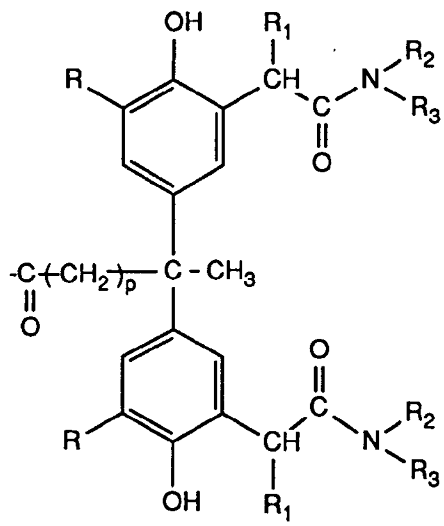

(IIIb)

steht, p 1 oder 2 und q eine ganze Zahl von 2 bis 12 bedeuten,

mit der Maßgabe daß

R nur einen Rest der Formel II darstellt, wenn $R_4$ nicht einer Gruppe der Formel III entspricht und das Molekül keinen Rest der Formel IIIa enthält.

**2.** Zusammensetzung nach Anspruch 1, worin
R $C_{1-18}$-Alkyl, Cyclohexyl, Phenyl, Benzyl oder ein Rest der Formel II ist,
$R_1$ Wasserstoff, Methyl, Ethyl oder Phenyl darstellt,
Y für eine direkte Bindung, Methylen oder Schwefel steht,
$R_2$ und $R_3$ unabhängig voneinander Wasserstoff, $C_{1-18}$-Alkyl, Cyclohexyl, Benzyl, Phenyl, mit $C_{1-12}$-Alkyl subtituiertes Phenyl, Naphthyl, -$(CH_2)_x$CO-$R_6$, $CH_2CH_2OH$ oder einen Rest der Formel

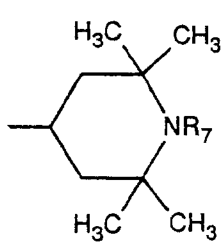

darstellen, oder $R_2$ -$NHR_{14}$ ist und $R_3$ die vorstehend genannten Bedeutungen hat, oder $R_2$ und $R_3$ zusammen mit dem N-Atom, an das sie gebunden sind, einen Piperidin-, Morpholin-, Piperazin- oder Hexamethyleniminring bilden,

oder, wenn $R_3$ = H und R nicht für eine Gruppe der Formel II steht, $R_2$ auch einen Rest-D-E bedeutet, worin E eine Gruppe der Formel IIa oder IIb ist, wobei R im Rest der Formel IIa, III, IIIa und IIIb $C_{1-18}$-Alkyl oder Cyclohexyl und

$R_8$ im Rest der Formel IIa für $C_{1-12}$-Alkyl oder Cyclohexyl stehen, y 0 bis 6 bedeutet,

$R_6$ Wasserstoff oder $C_{1-4}$-Alkyl bedeutet,

$R_7$ Wasserstoff, $C_{1-4}$-Alkyl oder CO-$C_{1-4}$-Alkyl ist,

$R_4$ Wasserstoff, $C_{1-12}$-Alkyl, Cyclohexyl, Phenyl, -$C_nH_{2n}CO_2R_{11}$ oder

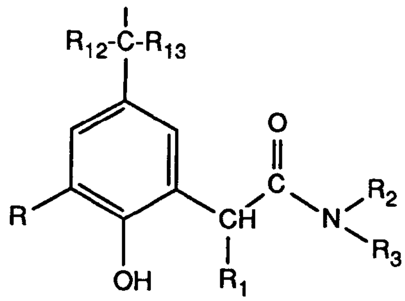

(III)

bedeutet,

$R_{12}$ und $R_{13}$ unabhängig voneinander Wasserstoff, $C_{1-4}$-Alkyl, Phenyl oder Cyclohexyl bedeuten oder, wenn $R_{12}$ Methyl ist, $R_{13}$ auch für

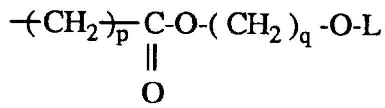

oder

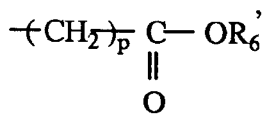

steht, q eine ganze Zahl von 2-6 darstellt und $R_6'$Wasserstoff oder $C_{1-18}$-Alkyl darstellen.

**3.** Zusammensetzungen nach Anspruch 2, worin

R $C_{1-18}$-Alkyl oder einen Rest der Formel II,

$R_1$ Wasserstoff oder Phenyl,

Y eine direkte Bindung und

$R_2$ Wasserstoff, $C_{1-20}$-Alkyl, Benzyl, Phenyl, Amino oder ein Rest der Formel IIIc

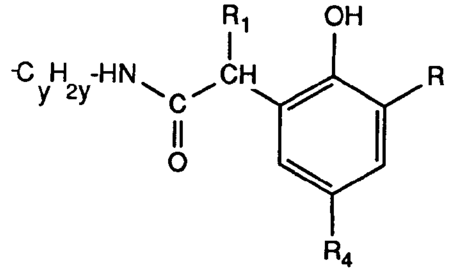

(IIIc)

oder

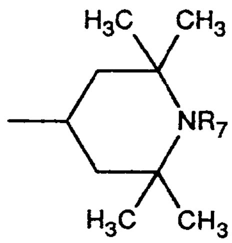

sind,
$R_4$ Wasserstoff, $C_{1-6}$-Alkyl oder eine Gruppe der Formel III bedeutet, worin $R_{12}$ und $R_{13}$ unabhängig voneinander Wasserstoff oder $C_{1-4}$-Alkyl sind sowie, wenn $R_{12}$ Methyl ist,
$R_{13}$ auch für $-(CH_2)_2COOCH_3$ steht,
$R_7$ Wassertoff, $C_{1-4}$-Alkyl oder -CO-$C_{1-4}$-Alkyl,
$R_3$ Wasserstoff und y 2- 12 bedeuten.

**4.** Zusammensetzungen nach Anspruch 3, worin
R $C_{1-6}$-Alkyl ist,
$R_1$ Wasserstoff oder Phenyl darstellt,

$R_2$ Wasserstoff, $C_{1-20}$-Alkyl, $-NH_2$, einen Rest der Formel

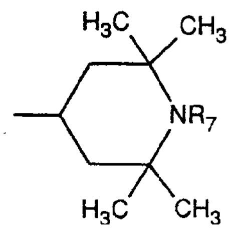

oder einen
Rest der Formel IIIc darstellen, worin y 2-10 ist, $R_7$ Wasserstoff, $CH_3$ oder $-COCH_3$ ist,
$R_4$ und $R_8$ Wasserstoff oder $C_{1-6}$-Alkyl bedeuten und $R_3$ für Wasserstoff steht.

**5.** Zusammensetzung nach Anspruch 1, worin das organische Material ein Schmierstoff, eine Metallbearbeitungsflüssigkeit, eine Hydraulikflüssigkeit oder ein natürliches oder (halb)synthetisches, insbesondere ein synthetisches Polymer ist.

**6.** Zusammensetzung nach Anspruch 5, worin das organische Material ein Schrnierstoff ist.

**7.** Zusammensetzung nach Anspruch 6, worin das organische Material ein thermoplastischer Kunststoff oder ein Elastomer ist.

**8.** Zusammensetzung nach Anspruch 7, worin das organische Material ein Polyolefin ist.

**9.** Zusammensetzung nach Anspruch 1, die zusätzlich weitere Stabilisatoren wie Antioxidantien, Lichtschutzmittel und/oder Verarbeitungsstabilisatoren (Hitzestabilisatoren) enthält.

**10.** Verbindungen der Formel I

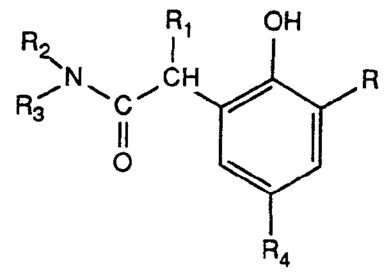

(I)

worin R Wasserstoff, $C_{1-24}$-Alkyl, $C_{5-12}$-Cycloalkyl, Phenyl, Phenyl-$C_{1-4}$-alkyl oder einen Rest der Formel II

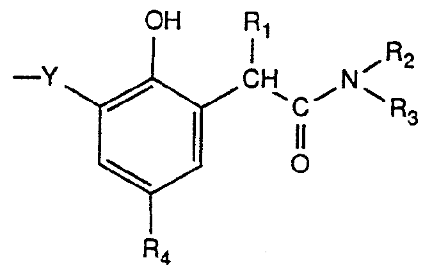

(II)

bedeutet,

Y für $-CHR_5-$, Schwefel oder eine direkte Bindung steht,

$R_1$ Wasserstoff, $C_{1-4}$-Alkyl, Phenyl oder mit Nitro, Chor, fluor, $C_{1-8}$-Alkyl oder/und Methoxy substituiertes Phenyl,

$R_5$ Wasserstoff, $C_{1-8}$-Alkyl oder Phenyl,

$R_2$ und $R_3$ unabhängig voneinander Wasserstoff, $C_{1-20}$-Alkyl, $C_{5-12}$-Cycloalkyl, Benzyl, Phenyl, mit $C_{1-18}$-Alkyl substituertes Phenyl, $C_{2-4}$-Hydroxyalkyl, Naphthyl, $(CH_2)_x-COR_6$,

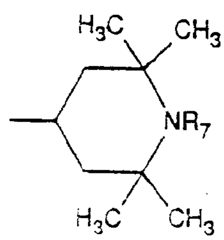 oder

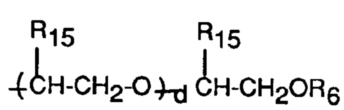

oder zusammen mit dem N-Atom, an das sie gebunden sind, einen 5-7-gliedrigen heterocyclischen Ring bedeuten,

oder $R_2$ $-NHR_{14}$ ist und $R_3$ die vorstehend genannten Bedeutungen hat,

$R_{14}$ Wasserstoff, $C_{1-12}$-Alkyl, Phenyl oder mit $C_{1-4}$Alkyl substituiertes Phenyl ist,

$R_{15}$ Wasserstoff, $C_{1-4}$-Alkyl oder Phenyl bedeutet,

d eine ganze Zahl von 1 bis 18 und x eine ganze Zahl von 1 bis 5 ist,

$R_6$ Wasserstoff oder $C_{1-20}$-Alkyl bedeutet,

$R_7$ Wasserstoff, $C_{1-8}$-Alkyl, $C_{1-12}$-Alkoxy oder $CO-R_9$ ist,

oder, wenn $R_3$ Wasserstoff ist und R nicht für eine Gruppe der Formel II steht,

$R_2$ auch einen Rest -D-E bedeutet, wobei -D- den Formeln

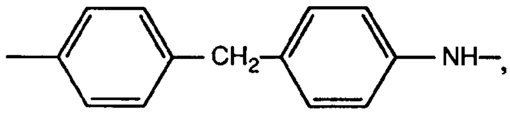 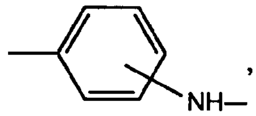 , $-C_yH_{2y}-NH-$,

$-(CH_2-CH_2-NH)_2-$ oder 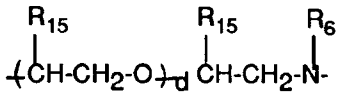

und E den Formeln

(IIa) 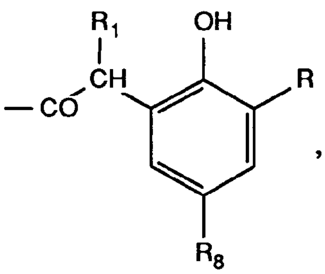 , 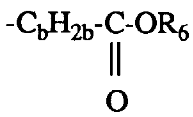 oder 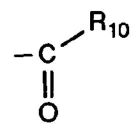 (IIb)

entsprechen und die

Reste E an die NH-Gruppen der Reste -D- gebunden sind, b für eine ganze Zahl von 0 bis 2 und y für eine ganze Zahl von 0 bis 12 stehen,

$R_4$ Wasserstoff, $C_{1-20}$-Alkyl, $C_{5-12}$-Cycloalkyl, Benzyl, Phenyl, mit $C_{1-4}$-Alkyl substituiertes Phenyl, $C_{1-4}$-Alkoxy, $-C_nH_{2n}CO_2R_{11}$ oder

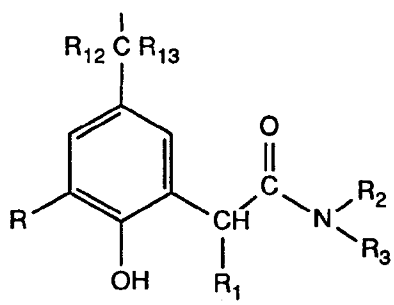

(III)

ist,

$R_{11}$ Wasserstoff, $C_{1-18}$-Alkyl, $-C_zH_{2z}$-A oder $-CH_2-C(CH_2A)_3$ bedeutet,

A für

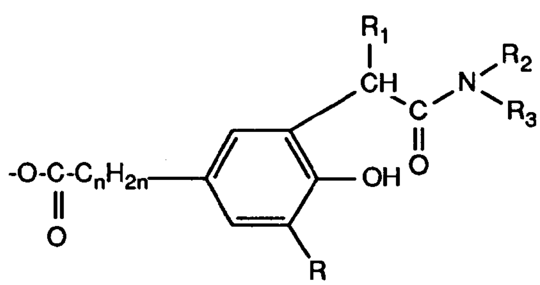

(IIIa)

und z für eine ganze Zahl von 2 bis 12 stehen, n die Werte 0, 1 oder 2 annimmt, $R_{12}$ und $R_{13}$ unabhängig voneinander Wasserstoff, $C_{1-8}$Alkyl, Phenyl oder $C_{5-12}$-Cycloalkyl bedeuten oder, wenn $R_{12}$ Methyl ist, $R_{13}$ auch für

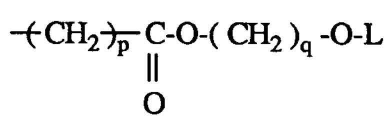 oder 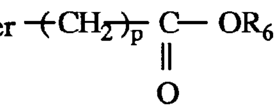

steht,
L für einen Rest der Formel

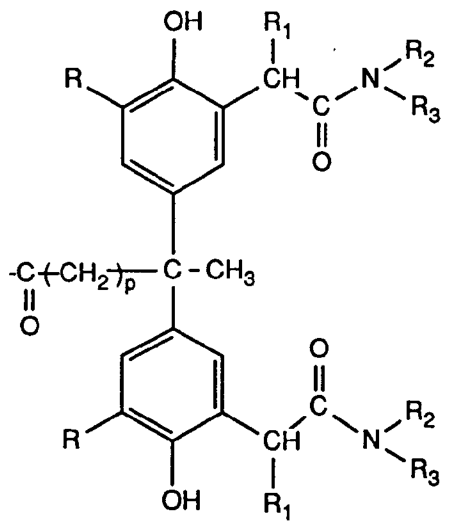

(IIIb)

steht, p 1 oder 2 und q eine ganze Zahl von 2 bis 12 bedeuten,
$R_8$ Wasserstoff, $C_{1-12}$-Alkyl oder $C_{5-12}$-Cycloalkyl bedeutet,
$R_9$ Phenyl oder $C_{1-8}$-Alkyl ist und
$R_{10}$ $C_{1-24}$-Alkyl oder Phenyl ist,
mit der Maßgabe daß
R nur einen Rest der Formel II
darstellt, wenn $R_4$ nicht einer Gruppe der Formel III entspricht und das Molekül keinen Rest der Formel IIIa enthält, sowie mit der weiteren Maßgabe, daß $R_2$ nicht Wasserstoff, $C_{3-12}$-Alkyl, Phenyl, Cyclopentyl oder Acetyl bedeutet, wenn $R_3$ Wasserstoff ist und gleichzeitig R für Wasserstoff oder Methyl steht.

**11.** Verbindungen nach Anspruch 10, worin
R $C_{1-18}$-Alkyl, Cyclohexyl, Phenyl, Benzyl oder ein Rest der Formel II ist,
$R_1$ Wasserstoff, Methyl, Ethyl oder Phenyl darstellt,
Y für eine direkte Bindung, Methylen oder Schwefel steht,
$R_2$ und $R_3$ unabhängig voneinander Wasserstoff, $C_{1-18}$-Alkyl, Cyclohexyl, Benzyl, Phenyl, mit $C_{1-12}$-Alkyl substituiertes Phenyl, Naphthyl, $-(CH_2)_xCO-R_6$, $CH_2CH_2OH$ oder einen Rest der Formel

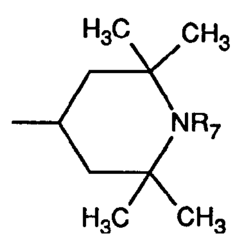

darstellen, oder $R_2$ $-NHR_{14}$ ist und $R_3$ die vorstehend genannten Bedeutungen hat, oder $R_2$ und $R_3$ zusammen mit dem N-Atom, an das sie gebunden sind, einen Piperidin-, Morpholin-, Piperazin- oder Hexamethyleniminring bilden,

oder, wenn $R_3$ = H und R nicht für eine Gruppe der Formel II steht, $R_2$ auch einen Rest -D-E bedeutet, worin E eine Gruppe der Formel IIa oder IIb ist, wobei R im Rest der Formel IIa, III, IIIa und IIIb $C_{1-18}$-Alkyl oder Cyclohexyl und
$R_8$ im Rest der Formel IIa für $C_{1-12}$-Alkyl oder Cyclohexyl stehen, y 0 bis 6 bedeutet,
$R_6$ Wasserstoff oder $C_{1-4}$-Alkyl bedeutet,
$R_7$ Wasserstoff, $C_{1-4}$-Alkyl oder CO-$C_{1-4}$-Alkyl ist,
$R_4$ Wasserstoff, $C_{1-12}$-Alkyl, Cyclohexyl, Phenyl, $-C_nH_{2n}CO_2R_{11}$ oder

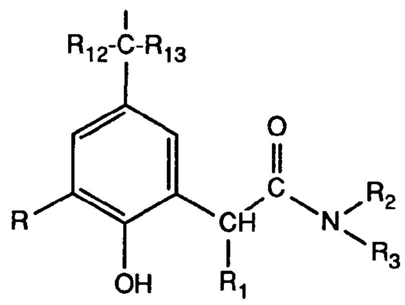

(III)

bedeutet,
$R_{12}$ und $R_{13}$ unabhängig voneinander Wasserstoff, $C_{1-4}$-Alkyl, Phenyl oder Cyclohexyl bedeuten oder, wenn $R_{12}$ Methyl ist, $R_{13}$ auch für

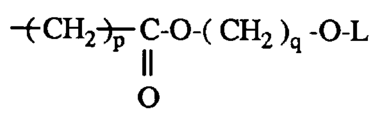

oder

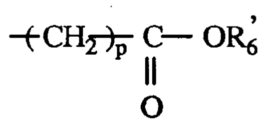

steht, q eine ganze Zahl von 2-6 und $R_6'$Wasserstoff oder $C_{1-18}$-Alkyl darstellen.

**12.** Verbindungen nach Anspruch 11, worin R $C_{1-18}$-Alkyl oder ein Rest der Formel II,
$R_1$ Wasserstoff oder Phenyl,
Y eine direkte Bindung,
$R_2$ Wasserstoff, $C_{1-20}$-Alkyl, Benzyl, Phenyl, Amino oder ein Rest der Formel IIIc

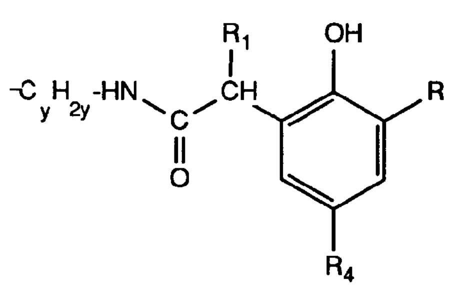

(IIIc)

oder

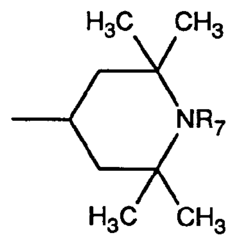

sind,
$R_4$ Wasserstoff, $C_{1-6}$-Alkyl oder eine Gruppe der Formel III bedeutet, worin $R_{12}$ und $R_{13}$ unabhängig voneinander Wasserstoff oder $C_{1-4}$-Alkyl sind sowie, wenn $R_{12}$ Methyl ist,
$R_{13}$ auch für $-(CH_2)_2COOCH_3$ steht,
$R_7$ Wasserstoff, $C_{1-4}$-Alkyl oder $-CO-C_{1-4}$-Alkyl,
$R_3$ Wasserstoff und y 2-12 bedeuten.

**13.** Verbindungen nach Anspruch 12, worin
R $C_{1-6}$-Alkyl ist,
$R_1$ Wasserstoff oder Phenyl darstellt,
$R_2$ Wasserstoff, $C_{1-20}$-Alkyl, $-NH_2$, einen Rest der Formel

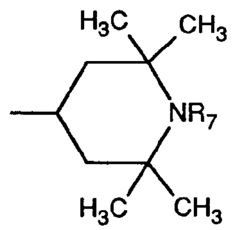

oder einen
Rest der Formel IIIc darstellen, worin
y 2-10 ist, $R_7$ Wasserstoff, $CH_3$ oder $-COCH_3$ ist,
$R_4$ und $R_8$ Wasserstoff oder $C_{1-4}$-Alkyl bedeuten und $R_3$ für Wasserstoff steht.

**14.** Verwendung von Verbindungen der Formel I gemaß den Ansprüchen 1 und 10 zum Stabilisieren organischen Materials gegen oxidativen, thermischen und/oder actinischen Abbau.

**15.** Verwendung von Verbindungen gemäß Anspruch 14 als Stabilisatoren in Schmierstoffen, Metallbearbeitungs- und Hydraulikflüssigkeiten sowie in synthetischen, natürlichen oder halbsynthetischen Polymeren.

**16.** Verfahren zum Stabilisieren organischen Materials, dadurch gekennzeichnet, daß man diesem Material als Stabilisatoren Verbindungen der Formel I nach den Ansprüchen 1 bzw. 10 zusetzt bzw. auf diese aufbringt.

**17.** Verfahren nach Anspruch 16 zum Stabilisieren von Schmierstoffen, Metallbearbeitungs- und Hydraulikflüssigkeiten sowie natürlichen, synthetischen oder halbsynthetischen Polymeren.

Europäisches Patentamt

# EUROPÄISCHER RECHERCHENBERICHT

Nummer der Anmeldung

EP 91 81 0593
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | GB-A-1 154 189 (FARBENFABRIKEN BAYER AG.)<br>* Beispiele 6,11,12 *<br>--- | 10,11 | C07C235/34<br>C07C243/32<br>C07D295/182<br>C09K15/22<br>C09K15/30<br>C10M133/16<br>C10M133/40<br>C08K5/20<br>C08K5/22<br>C08K5/3435 |
| X | CHEMICAL ABSTRACTS, vol. 75, no. 7, Columbus, Ohio, US;<br>abstract no. 47864Y,<br>ELGALA, A. M.; MAIER, R. H.: 'Effect of ethylenediamine di(O-hydroxyphenylacetic acid) application to soil columns on the distribution of certain nutrient elements in the water soluble, acid soluble, and exchangeable forms'<br>Seite 266 ;<br>* Zusammenfassung *<br>& PLANT SOIL<br>Bd. 34, Nr. 3, 1971,<br>Seiten 607 - 617;<br>& Chemical Abstracts 8th Collective Index<br>page 145S<br>Acetamide, N,N'-ethylene bis[2-(o-hydroxyphenyl)-, monosodium salt<br>--- | 10,11 | |
| | | | **RECHERCHIERTE SACHGEBIETE (Int. Cl.5 )** |
| X | EP-A-0 281 471 (ADIR ET CIE)<br>* Beispiel 6A *<br>--- | 10,11 | C07C<br>C07D |
| X | EP-A-0 253 665 (MINNESOTA MINING AND MFG. CO)<br>* page 8 : Compound 4 *<br>* Beispiel 3 *<br>--- | 10,11 | |
| X | EP-A-0 153 476 (BASF AG.)<br>* Ansprüche; Beispiele 1-3 *<br>--- | 10,11 | |
| D,X | DE-A-1 959 898 (GEIGY J.R. A.-G.)<br>* Beispiel 6 * | 10 | |
| A | * Beispiele 1-5,7,8 *<br>---<br>-/-- | | |

Der vorliegende Recherchenbericht wurde für alle Patentansprüche erstellt

| Recherchenort | Abschlußdatum der Recherche | Prüfer |
|---|---|---|
| DEN HAAG | 20 NOVEMBER 1991 | PAUWELS G.R.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 91 81 0593
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X<br><br><br><br><br><br><br><br><br><br>D | CHEMICAL ABSTRACTS, vol. 70, no. 17, 28. April 1969, Columbus, Ohio, US; abstract no. 77540D, LANGE, JERZY; URBANSKI, TADEUSZ: 'Antitubercular compounds. XLII. Preparation and antibacterial properties of certain derivatives of isomeric hydroxyphenylacetic acids' Seite 300 ; * Zusammenfassung *<br>& DISS. PHARM. PHARMACOL. Bd. 20, Nr. 6, Seiten 607 - 613; | 10,11 | |
| X | ARZNEIMITTEL FORSCHUNG. Bd. 26, Nr. 10, 1976, AULENDORF DE Seiten 1837 - 42; Brandau, K.; Keup, U.: 'Studies on the determination and induction of cholesterol 7.alpha.-hydroxylase' Summary: Spalte 4. | 10 | |
| | | | RECHERCHIERTE SACHGEBIETE (Int. Cl.5 ) |

Der vorliegende Recherchenbericht wurde für alle Patentansprüche erstellt

| Recherchenort | Abschlußdatum der Recherche | Prüfer |
|---|---|---|
| DEN HAAG | 20 NOVEMBER 1991 | PAUWELS G.R.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)